(19) Europäisches Patentamt European Patent Office Office européen des brevets

(11) **EP 4 156 202 A1**

(12) **EUROPEAN PATENT APPLICATION**
published in accordance with Art. 153(4) EPC

(43) Date of publication:
**29.03.2023 Bulletin 2023/13**

(21) Application number: **21808663.5**

(22) Date of filing: **20.05.2021**

(51) International Patent Classification (IPC):
*G16H 40/20* (2018.01)    *G06Q 10/06* (2012.01)
*G06N 20/00* (2019.01)    *G06N 3/08* (2006.01)

(52) Cooperative Patent Classification (CPC):
**G06N 3/08; G06N 20/00; G06Q 10/06; G16H 40/20**

(86) International application number:
**PCT/KR2021/006287**

(87) International publication number:
**WO 2021/235866 (25.11.2021 Gazette 2021/47)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **20.05.2020 KR 20200060548
09.12.2020 KR 20200171441**

(71) Applicant: **Seoul National University Hospital
Seoul 03080 (KR)**

(72) Inventors:
• **KIM, Joonghee**
  **Seongnam-si, Gyeonggi-do 13596 (KR)**
• **JEONG, Joo**
  **Seoul 06511 (KR)**
• **KIM, Dae Kon**
  **Seongnam-si, Gyeonggi-do 13482 (KR)**

(74) Representative: **V.O.**
  **P.O. Box 87930**
  **2508 DH Den Haag (NL)**

(54)    **METHOD AND SYSTEM FOR PREDICTING NEEDS OF PATIENT FOR HOSPITAL RESOURCES**

(57)    The embodiments relate to a method for predicting needs of a patient for hospital resources, and a system for carrying out same, the method comprising the steps of: generating numerical data per information type by encoding natural language data and structured data, which are in patient data recorded in language and digits; and, by applying the numerical data per information type to an artificial neural network, predicting a task corresponding to the needs of the patient for hospital resources.

FIG. 2

EP 4 156 202 A1

## Description

## Technical Field

The present invention relates to a technology for predicting needs of a patient for hospital resources, and more particularly, a method for predicting needs of a patient for hospital resources by processing natural language data and structured data indicating the condition of an emergency patient, and a system for performing the same.

## Background Art

Accurately identifying a patient's condition in emergency medical services (EMS) is an important factor that needs to be analyzed for the patient's prognosis. Currently, managers of emergency medical service directly read patient data and provide clues for needs of patients.

However, since emergency medical services must be provided 24 hours a day and 365 days a year, it is difficult to accurately predict the needs of patients when the managers' workload is increased.

Therefore, there is a need for a technology capable of predicting patient needs with performance comparable to that of a human manager for 24 hours a day and 365 days.

[Citation List]

[Patent Literature]

[Patent Literature 1]
Patent Publication No. 10-2009-0001551 (January 09, 2009)

## Disclosure

## Technical Problem

According to one aspect of the present invention, it is possible to provide a system for performing an operation of predicting needs of a patient for hospital resources by processing natural language data and structured data indicating a condition of an emergency patient.

In addition, it is possible to provide a method for predicting needs of a patient for hospital resources and a computer-readable recording medium on which the method is recorded.

## Solution to Problem

According to exemplary embodiments of the present invention, a method of predicting needs of a patient for hospital resources which is performed by a processor includes: generating numerical data per information type by encoding natural language data and structured data, which are in patient data recorded in language and digits; and, by applying the numerical data per information type to an artificial neural network, predicting a task corresponding to the needs of the patient for hospital resources, wherein the artificial neural network includes an embedding model for calculating an embedding matrix of the patient data based on the numerical data; and a decision model for determining a task to which the patient data belongs by receiving an intermediate data set including the embedding matrix of the patient data.

According to exemplary embodiments of the present invention, a system includes a data acquisition device configured to acquire patient data including natural language data and structured data describing a condition of a patient, which are recorded in language and digits; an encoding module configured to generate numerical data per information type by encoding the natural language data and the structured data in the patient data; a prediction module configured to predict a task corresponding to needs of the patient for hospital resources by applying the numerical data to an artificial neural network, wherein the artificial neural network includes an embedding model for calculating an embedding matrix of the patient data based on the numerical data; and a decision model for determining a task to which the patient data belongs by receiving an intermediate data set including the embedding matrix of the patient data.

## Advantageous Effects of the Invention

The method for predicting needs of a patient for hospital resources according to an aspect of the present invention may predict needs of the patient by analyzing natural language data and structured data included in patient data through a pre-trained artificial neural network.

The effects of the present disclosure are not limited to the aforementioned effects, and any other effects not mentioned herein will be clearly understood from the claims by those skilled in the art.

## Brief Description of Drawings

In order to more clearly describe the technical solutions of the embodiments of the present invention or the prior art, drawings necessary for the description of the embodiments are briefly introduced below. It should be understood that the following drawings are for the purpose of explaining the embodiments of the present specification and not for the purpose of limitation. In addition, some elements to which various modifications such as exaggeration and omission have been applied may be shown in the drawings below for clarity of description.

FIG. 1 is a block diagram of a system for performing an operation of predicting needs of a patient for hospital resources according to an embodiment of the present invention.

FIG. 2 is a conceptual diagram of an artificial neural network according to an embodiment of the present invention.

FIG. 3 is a conceptual diagram of an artificial neural network according to another embodiment of the present invention.

FIG. 4 is a flowchart of a method for predicting needs of a patient for hospital resources according to an embodiment of the present invention.

FIG. 5A is a diagram comparing the prediction performance of an artificial neural network of FIG. 2 and the prediction performance of a human expert according to an experimental example of the present invention.

FIG. 5B is a diagram comparing the prediction performance of an artificial neural network of FIG. 3 and the prediction performance of a human expert according to an experimental example of the present invention.

FIGS. 6A to 6D are diagrams illustrating samples of an attention map according to an experimental example of the present invention.

**Mode for Invention**

The terminology used herein is for the purpose of describing particular embodiments only and is not intended to be limiting of the invention. As used herein, the singular forms may be intended to include the plural forms as well, unless the context clearly indicates otherwise. It will be further understood that the terms "comprises" and/or "comprising," when used in this specification, specify the presence of stated features, regions, integers, steps, operations, elements, and/or components, but do not preclude the presence or addition of one or more other features, regions, integers, steps, operations, elements, and/or components.

Unless otherwise defined, all terms including technical and scientific terms used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

Hereinafter, embodiments of the present invention will be described in detail with reference to the drawings.

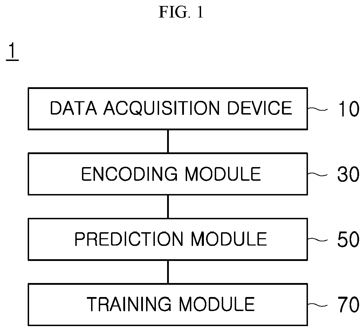

FIG. 1 is a block diagram of a system for performing an operation of predicting needs of a patient for hospital resources according to an embodiment of the present invention.

Referring to FIG. 1, a system (hereinafter, "prediction system" 1 ) for performing an operation of predicting needs of a patient for hospital resources may include a data acquisition device 10, an encoding module 30, and a prediction module 50. In some embodiments, the prediction system 1 may further include a training module 70.

The prediction system 1 according to the embodiments may be entirely hardware, entirely software, or partly hardware and partly software. For example, the system may collectively refer to hardware equipped with data processing capability and operating software for driving the same. As used herein, terms such as "unit," "module," "device," or "system" are intended to refer to a combination of hardware and software executed by the hardware. For example, the hardware may be a data processing device including a central processing unit (CPU), a graphic processing unit (GPU), or other processors. In addition, the software may refer to a running process, an object, an executable file, a thread of execution, a program, and the like.

The prediction system 1 may acquire patient data by means of a data acquisition device 10. The data acquisition device 10 may include a data input device, a data transmitter/receiver, an image input device, and/or a video input device. The prediction system 1 may acquire patient data in the form of text, images, or videos.

The patient data is data for describing a patient's condition. The patient may be an emergency patient, but is not limited thereto.

The patient's condition is expressed in one or more sentences. For example, patient data for an emergency patient may be expressed in a single sentence since the emergency room condition is urgent. The sentence consists of a plurality of words.

Hereinafter, for clarity of description, the present invention will be described in more detail based on patient data in which the patient's condition is expressed in a single sentence composed of a plurality of words.

The patient data may include data to be structured (hereinafter, referred to as "structured data") and natural language data. The structured data and/or natural language data may be expressed in a single sentence.

The structured data may include information that can be structured by being expressed in numerical or categorical form.

In an embodiment, the patient data may include measurement information as numerical data, and/or demographic information as categorical information.

The demographic information may include, for example, age and gender, but is not limited thereto.

The measurement information may include various measurement values acquired by measuring a patient's physical condition. In certain embodiments, the measurement information may include a measurement value for one or more measurement items including a pupil state, a systolic blood pressure (SBP), a diastolic blood pressure (DBP), a pulse, a respiratory rate, a body temperature, a consciousness level, and an initial $O_2$ saturation.

The natural language data is data other than the structured data in the patient data. Some or all of data handwritten by a medical staff may be used as natural language data. In certain embodiments, the natural language data may include one or more of current disease information, main symptom-related information, injury-related information, and history-related information. The current disease information may include history of present illness (HPI). The main symptom-related information may include chief complaints (CC). The injury-related information may include an injury summary. The history-related information is information about past medical or surgical history, and may include, for example, past medical history (PMH).

The natural language data may be classified into a first type of information and a second type of information. The first type of information among the natural language data is information that is considered more important in predicting needs of a patient. In certain embodiments, the first type of information in the natural language data may be information about a current disease. The reason for this is that HPI is the most commonly used data to determine needs of a patient.

Among the natural language data, the second type of information is information considered less important than the first type of natural language data in predicting needs of a patient needs. In certain embodiments, the second type of information of the natural language data may include some or all of the remaining information other than the first type of information among the natural language data.

The data acquisition device 10 supplies acquired patient data to the encoding module 30.

The encoding module 30 performs a pre-processing operation for converting patient data including natural language data and structured data into numerical data. This pre-processing operation is referred to as an encoding operation. Since only numerical data is allowed to be input to the artificial neural network, patient data is able to be input to the artificial neural network by the encoding module 30.

The encoding module 30 encodes natural language data and structured data to generate numerical data per information type. Generating the numerical data is performed through natural language processing operations.

Among patient data, natural language data and categorical data are recorded in language, so they are converted into character string values when simply converted into data. Since character string values are not allowed as input values to the artificial neural network, the encoding module 30 converts the patient data, that is, language data in an input sentence, into text data as character string data, and then converts the text data into numerical data.

A process of digitizing the text is performed through embedding processing, and the results of digitization of the text is acquired as an embedding vector. That is, the embedding vector indicates a result of digitizing corresponding text, and, for example, the word embedding indicates a result of digitizing text in a word unit. The word unit is referred to as a token, which means a commonly used word or a minimal unit of sentence decomposition, such as a morpheme or the like. The token may refer to each character in an extreme case.

In an embodiment, the encoding module 30 may output embedding data for some or all of text in a sentence in tokenization units.

The natural language data is tokenized and indexed by a natural language processing tool for a written language. For example, when processing Korean, all elements of a sentence are decomposed into minimum units (that is, tokens) such as morphemes (referred to as tokenization), and elements of a set of tokens acquired therefrom may be preprocessed by assigning their own unique numbers to the elements. However, in addition to this, subword segmentation (e.g., byte-pair encoding) or various other tokenization methods may also be used.

The tokenization unit may be specified as a word. Then, the tokenization is performed based on various language databases (e.g., Wikipedia-based in-house corpus dataset in case of Korean). The encoding module 30 acquires corpus data by segmenting the natural language data expressed in a sentence (e.g., crawling, etc.), and adds/removes/modifies punctuation marks, special characters, spaces, or the like in the corpus data and tokenizes the corpus data in units of words/morphemes/characters.

Through this natural language processing, the encoding module 30 identifies an information type of the patient data. The text data of the natural language data is classified into a first type of information or a second type of information among the natural language data. For example, a word corresponding to HPI in a single sentence is identified as the first type of natural language data.

An identifier indicating the first type may be associated with data of the first type of information among the natural language data.

As described above, the encoding module 30 generates text data for the language in the patient data through a natural language processing operation, and finally generates numerical data of the first type of information among the natural language data, that is, a first type of natural language embedding vector, and numerical data of the second type of information among the natural language data, that is, a second type of natural language embedding vector.

Also, when the categorical data is recorded in language, the encoding module 30 may encode the categorical data into numerical data from the acquired patient data. For example, the encoding module 30 may convert the categorical data into the numerical data by performing one-hot encoding on features of demographic information.

The encoding module 30 converts measurement information in the patient data into numerical data.

In an embodiment, the encoding module 30 may form a measurement matrix (or vector). The measurement matrix (or vector) is a matrix (or vector) consisting of measurement values for measurement items for a patient, and the positions of internal elements thereof indicate the measurement items.

Also, the encoding module 30 may be further configured to pre-process measurement information. In an embodiment, the encoding module 30 may encode the numerical data through a standardization process for removing the mean from the features of the numerical data and/or performing scaling according to a unit variance. Then, a measurement matrix (or vector) is formed with the standardized values.

In this way, numerical data per information type is generated through natural language processing. The encoding module 30 generates a first type of natural language embedding vector calculated by embedding text data of current disease information acquired through the natural language processing, a second type of natural language embedding vector calculated by embedding text data of the remaining information of the natural language data acquired through the natural language processing, an embedding vector of demographic information calculated by embedding text data of the demographic information acquired by the natural language processing of the demographic information among the structured data, and/or numerical data (e.g., a measurement matrix) of the measurement information converted through the natural language processing, and supply a data set including at least one of these numerical data to the prediction module 50.

In an embodiment, when the first type of information of the natural language data consists of a plurality of words, the encoding module 30 may also calculate a first type of natural language embedding vector for each word constituting the first type of information. Then, the numerical data of the first type of information (e.g., HPI) is supplied to the prediction module 50 as a set of a plurality of word embedding vectors.

Also, the encoding module 30 may generate a single embedding vector for information describing the patient in context. The single embedding vector may consist of information of a type different from the first type of information, for example, the second type of natural language embedding vector, the embedding vector of demographic information and/or numerical data of the measurement information. This single embedding vector is referred to as a contextual embedding vector. The information of the second type of information, or the information of the categorical data and the information of the numerical data in the natural language data is considered in predicting the needs of a patient through the contextual embedding vector.

The prediction module 50 may predict the needs of a patient using the first type of natural language embedding vector (e.g., a set of a plurality of word embedding vectors) and the contextual embedding vector.

The prediction module 50 predicts the needs of a patient for hospital resources by applying data, obtained by encoding the patient data, to the artificial neural network. The prediction module 50 may apply numerical data per information type, such as an embedding vector of an HPI word in a sentence, to the artificial neural network.

The prediction module 50 may perform an operation similar to that of a user (e.g., a medical staff or an emergency manager) who predicts the needs of a patient by directly reading the patient data to predict the needs of a patient, by using the artificial neural network. For example, the prediction module 50 may perform an operation of briefly checking a portion of natural language data such as main symptom items and demographic information in the patient data; an operation of reading HPI in detail; and an operation of interpreting various measurement values (e.g., vital signs, etc.) in order to predict the needs of a patient. In addition, in the case of predicting a specific event (e.g., the case of predicting an event in which a patient stays in the emergency room), the prediction module 50 may further perform an operation of referring back to patient data and re-analyzing the patient data while focusing on specific parts of text relevant to a corresponding event. At least some of these operations may be modeled as an operation of the artificial neural network.

In an embodiment, the artificial neural network may include: an embedding model E for calculating an embedding matrix (or vector) of the patient data based on the numerical data; and a decision model D for determining a task to which the patient data belongs. As in the above assumption, when the patient data is a single sentence, the embedding model E calculates a sentence embedding matrix. Then, the decision model D determines a task to which the sentence belongs to predict the patient needs.

The prediction module 50 may input the first type of natural language embedding vector (e.g., a set of a plurality of word embedding vectors) to the embedding model E, or input the first type of natural language embedding vector and the contextual embedding vector to the embedding model E to calculate an embedding vector for a sentence (that is, patient data).

To this end, the embedding model E may include one or more hidden layers that extract features of input data to calculate a hidden state vector. When a set of numerical data including a contextual embedding vector is input to the embedding model E, an embedding vector for a sentence is calculated. As described above, when a corresponding sentence includes a plurality of words, the embedding model E may calculate a sentence embedding matrix.

The embedding model E may have an RNN-based structure. In an embodiment, the embedding model E may have a unidirectional or bidirectional gated recurrent unit (GRU)-recurrent neural network (RNN)-based structure. Then, the embedding model E includes a unidirectional or bidirectional GRU-based hidden layer.

The embedding model E may be modeled as at least two structures depending on a process of inputting and processing context information, that is, a contextual embedding vector.

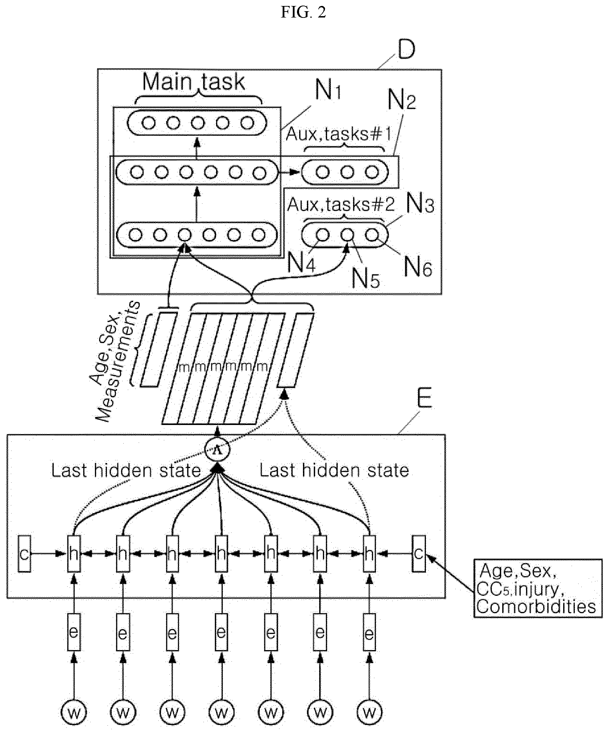

FIG. 2 is a conceptual diagram of an artificial neural network according to an embodiment of the present invention.

Referring to FIG. 2, a contextual embedding vector may be overwritten on initial hidden states of the GRU. In the above embodiment, embedding vectors e of tokens, which are the results of pre-processing on natural language information, are input to the embedding model E.

In the artificial neural network of FIG. 2, a contextual embedding vector c is specified as the initial hidden state of the embedding model E. When a sentence in the patient data is tokenized, the embedding vectors e of each token may be sequentially input to the embedding model E to calculate a sentence embedding matrix.

In the unidirectional GRU, the input of the embedding vectors e of the tokens is made only in the forward direction, and only one output vector is generated at each input timestep.

In the bidirectional GRU, in addition to the above-mentioned process, the embedding vectors e are input in the reverse direction from the last token of the input sentence, and two vectors (output vector obtained in the forward operation process and output vector obtained in the reverse operation process) of each token obtained in this forward/reverse operation processes are concatenated as one vector, which is processed as an input to the next hidden layer of the network.

Specifically, in the artificial neural network of FIG. 2, first, a contextual embedding vector c is set as an initial hidden state vector of the embedding model E.

Thereafter, first type of natural language embedding vectors e obtained by encoding the first type of natural language data w by the encoding module 30 is sequentially input to the embedding model E.

Whenever the embedding vector e is input in the forward/reverse direction one by one, each hidden layer corresponding to the forward/reverse direction is updated based on the input value, and a hidden state vector of the updated hidden layer (or the result of applying additional calculations to the hidden state vector) is calculated. Among the hidden state vectors, a state vector that is finally calculated in a forward or reverse direction may be referred to as a last hidden state vector.

When all the hidden layers of the embedding model E are passed, final hidden state vectors are calculated. The final state vectors are used to predict tasks.

In addition, the embedding model E is further configured to form an attention matrix A based on an attention weight. The attention matrix A is used together with the output matrix H of the sentence based on the final hidden state for more efficient task prediction.

In the artificial neural network, the embedding model E functions as an encoder and the decision model D functions as a decoder. The attention weight is used to refer once again to the entire input sentence of the encoder at every timestep at which the decoder predicts a task associated with a sentence.

However, the decision model D analyzes the input data while paying more attention to on an input word that is related to the task to be predicted at a corresponding timestep, rather than referring all input words (or vectors) at the same rate.

To this end, the embedding model E may form an output matrix H of the sentence based on the final hidden state vectors. The output matrix H is a matrix consisting of final hidden state vectors, and may be referred to as a hidden matrix H.

For example, the embedding model E forms a hidden matrix H having the form of $n \times 2 \times 1$ by combining hidden state vectors $h_s$ in the $d_h$ dimension of the hidden layer at each timestep. Here, n denotes a timestep and 1 denotes a length of the hidden layer in one direction. Since the hidden layer is typically composed of a bidirectional GRU network, the hidden matrix H has 2l columns. In the unidirectional GRU structure, the hidden matrix H has the form of $n \times l$.

Then, the embedding model E calculates an attention weight a for the hidden matrix H in order to form an attention matrix A. The attention weight a for the hidden matrix H is expressed by the following equation.

[Equation 1]

$$a = \text{softmax}(w_{s2}\tanh(W_{s1}H^{T}))$$

where $W_{s1}$ is a weight matrix having the form $d_a \times 2ld_h$, and $W_{s2}$ is a vector of size da. Here, da is a hyper parameter value and may be optimized through a training process.

To obtain a representation vector m of HPI, the hidden matrix H consisting of the hidden state vectors hs is summed into a weight a.

When a single sentence includes multiple expressions, the size matrix of $W_{s2}$ may be expanded in the form of $r \times d_a$. Here, r represents the number of expressions included in a single sentence. When the size matrix of $W_{s2}$ is expanded, the weight vector a forms the attention matrix A.

By multiplying the attention matrix A by the hidden matrix H, the embedding matrix M of the sentence may be calculated. The embedding matrix M of the sentence includes an embedding vector m for each expression. Using the attention matrix A, the artificial neural network may analyze (or learn) multiple expressions from a single sentence.

The value output from the embedding model E is supplied to the decision model D and used to predict the needs of a patient. The value output from the embedding model E includes a sentence embedding matrix M, and/or a hidden state vector such as a final hidden state vector.

The decision model D includes a fully connected layer. The fully connected layer may be formed of a plurality of layers. For example, the decision model D may consist of a fully connected layer of double or triple layers.

The parameters of the fully connected layer are trained to predict a preset task. A task is a work to be performed through the artificial neural network, and the needs of a patient may correspond to one or more tasks.

The task to be performed by the decision model D may be a main task, a first auxiliary task group, and/or a second auxiliary task. Each task may contain one or more items as a class. When data input to the decision model D is classified into a specific class, a categorical task including the classified specific class is determined as needs of a corresponding patient.

A target variable indicating a task may be extracted from an electronic health record (EHR) database. Tasks may be grouped into multiple categories. The task extracted from the EHR database are classified into a plurality of categories and reflected in the artificial neural network.

The main task may include a task class related to an expert's diagnosis of a patient's condition. For emergency room patients, the main task may include one or more of task classes including, for example, hospital admission, endotracheal intubation, mechanical ventilation, vasopressor infusion, cardiac catheterization, surgery, intensive care unit (ICU) admission, cardiac arrest within 24 hours after emergency room (ED) arrival but is not limited thereto.

The first auxiliary task may include a task class related to diagnosing a disease for a patient. For example, the first auxiliary task may include a task class of the patient's diagnostic disease name code. The diagnostic disease name code may be a code known to those skilled in the art, such as a Korean Classification of Disease (KCD) database. The first auxiliary task may include a plurality of disease names indicated by the code as a task class.

The second auxiliary task may include a task class related to the patient's treatment result. For example, the second auxiliary task may include one or more of task classes including hospital discharge, ward admission, intensive care unit admission, operating room (OR) transfer, and death.

In certain embodiments, the operation of predicting the needs of a patient based on the patient data in the decision model D refers to an operation of classifying a corresponding patient into a plurality of needs categories (e.g., presence/absence or two or more categories) based on the patient data (or a sentence) and may be implemented as multi-label binary classification and/or multinomial classification.

Then, the determining of the main task is a multiple binary prediction question, and when the correct answer of the multiple binary prediction question is answered, it is predicted to have a need corresponding to the correct answer. For example, the needs of a corresponding patient are predicted by answering the correct answer to the multiple binary prediction question, such as a 'yes/no' judgment about whether to be admitted to a hospital.

For the prediction of such needs, the decision model D may use the output value of the embedding model E and/or the output value of the encoding module 30 (e.g., encoded structured data). The decision model D may use data differently per task.

In an embodiment, the decision model D may use a value output from the embedding model E and a value preprocessed by the encoding module 30 to predict the main task and/or the first auxiliary task. The value output from the embedding model E may include a sentence embedding vector m, a sentence embedding matrix M, a hidden state vector and/or a final hidden state vector. The preprocessed value may include a measurement matrix or numerical data of demographic information as a result of encoding structured data.

For example, as shown in FIG. 2, a value output from the embedding model E such as the sentence embedding vector m, the sentence embedding matrix M, and the final hidden state vector and the encoding result of the structured data are input into the decision model D to predict the main task and the first auxiliary task.

The prediction operation for the second auxiliary task is performed in the same manner as the prediction operation for the main task and the first auxiliary task, but only a value output from the embedding model E may be used as an input value. This corresponds to a method for more selectively improving the performance of the embedding model.

To this end, the decision model D may consist of networks per tasks.

In an embodiment, the decision model D may include a first network N1 for determining a main task, and/or a second network N2 for determining a first auxiliary task. Here, the first network N1 and the second network N2 receive a value output from the embedding model E as an input, or a value output from the embedding model E and a final hidden state vector together as inputs, and determine the patient's condition and/or the patient's disease name.

The network N1 and the network N2 may include a shared hidden layer having the same input as each other. Then, the final output of a shared network is used to determine the main task or the first auxiliary task.

In addition, the decision model D may include a third network N3 for determining a second auxiliary task. In some embodiments, the network N3 may include a plurality of sub-networks. For example, as shown in FIG. 2, the network N3 may include a plurality of sub-networks N4, N5, and N6.

The third network (or plurality of sub-networks) is configured to use only the value output from the embedding model E to determine the second auxiliary task. For example, the networks N4, N5, and N6 performing a group of second auxiliary tasks may receive only values output from the embedding model E (sentence embedding vector m, sentence embedding matrix M, final hidden state vector, and the like) as input, and there is no hidden layer shared with other networks N1 and N2.

The processing results of these networks N4, N5, and N6 are used to perform classification into 5 classes for whether it corresponds to hospital discharge, whether it corresponds to ward admission, whether it corresponds to intensive care unit admission, whether it corresponds to operating room transfer, and whether it corresponds to death. As a result, the decision model D may determine the second auxiliary task to which an input sentence belongs.

Additionally, the prediction module 50 is configured to input an embedding matrix of a sentence output from the embedding model €, a final hidden state vector, and structured data through or not through the shared network of the networks N1 and N2. Then, the numerical data of the structured data may be directly/indirectly input to the networks N1 and N2.

Meanwhile, the values output from the embedding model E (sentence embedding vector m, sentence embedding matrix M, final hidden state vector, etc.) are input to the networks N4, N5, and N6 for determining the second auxiliary task.

The decision model D may calculate a probability that the sentence of an intermediate data set belongs to a certain class in a corresponding categorical task based on the output of a network per categorical task. For example, a probability for a task may be calculated based on an intermediate output or a final output of the shared network, respectively. The decision model D may include a Softmax function to calculate a probability from the output of a fully connected layer, but is not limited thereto.

When a task is determined, it is predicted that needs corresponding to the determined task are required of the patient. For example, when an intermediate data set representing a specific sentence is input to the decision model D and hospital admission is output among the main tasks, It is predicted that the corresponding patient has needs for hospital admission as the main task.

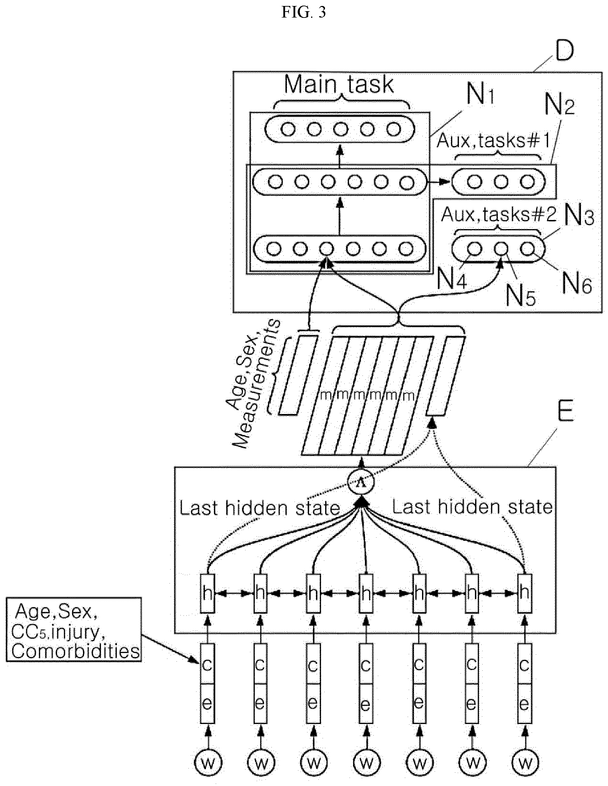

FIG. 3 is a conceptual diagram of an artificial neural network according to another embodiment of the present invention.

Since the artificial neural network of FIG. 3 is similar to the artificial neural network of FIG. 2, the differences will be mainly described.

Referring to FIG. 3, the embedding matrix (or vector) of the sentence may be calculated by combining contextual embedding vectors c with embedding vectors of a word and sequentially inputting the same into the embedding model E in one direction or both directions.

The contextual embedding vectors c are respectively combined with the first type of natural language embedding vectors e and input to the embedding model E. For example, as shown in FIG. 3, the contextual embedding vectors c are combined with a plurality of word embedding vectors e of HPI, respectively, and the combined vector is input to a unidirectional or bidirectional GRU-based hidden layer.

A process after data input into the embedding model E, such as the process of calculating the output matrix H by calculating the final hidden state vector and finally calculating the sentence embedding matrix M, is the same as that of FIG. 2, and a detailed description thereof is omitted.

The prediction system 1 may use an artificial neural network trained by an internal component (e.g., the training module 70), or an artificial neural network trained in advance through an external processor.

The artificial neural networks of FIGS. 2 and 3 are trained (e.g., by the training module 70) using a training data set consisting of multiple training samples.

Each training sample in the training data set includes structured data and natural language data of a training patient. For example, each training sample may include at least one of age, sex, main symptom-related information (e.g., CC), injury-related information (e.g., injury summary), historical information (e.g., PMH), current disease information (e.g., HPI), pupil state including size and reflex, systolic blood pressure (SBP, mmHg), diastolic blood pressure (DBP, mmHg), pulse (PR, beats per minute), respiratory rate (PR, breaths per minute), body temperature (BT, °C), level of consciousness (e.g., AVPU), and initial $O_2$ saturation (SpO$_2$ in pulse oximetry, %).

In the sentence in which the condition of the training patient is recorded, natural language data is arranged as text data of natural language-processed words by correcting lowercase letters, spaces, or the like through natural language processing. A training data set including the arranged text data is used to train the artificial neural network.

The parameters of the artificial neural network are trained to minimize a loss function.

The loss function includes a number of terms. In an embodiment, the loss function includes a cross entropy loss term (ECL) and a penalty term (P). The ECL is the weighted sum of the cross entropy losses of the network for the task. For example, when the decision model D includes a network N1 for a main task, a network N2 for a first auxiliary task, and a network N3 for a second auxiliary task, the first term consists of a weighted sum of cross entropy losses of the networks N1, N2, and N3.

In an embodiment, the weight distribution for the main task and the weight distribution for all auxiliary tasks are specified as 1:1. Here, the weight distribution for each auxiliary task among all auxiliary tasks is redistributed according to the number of networks per auxiliary category task. In the above example, when the decision model D includes networks for six categorical tasks, the number of networks of all auxiliary tasks including the first and second auxiliary tasks is 5, so that the weight distribution of the variables for each auxiliary task may be specified as 0.1, respectively.

The error of the first auxiliary task is used to improve the generalization of all of the networks of the decision model D. On the other hand, the error of the second auxiliary task is used to improve the generalizability of the Bi-GRU network in the artificial neural network.

The penalty term (P) in the loss function is expressed by the following equation.

[Equation 2]

$$P = \left\| AA^T - I \right\|_F^2$$

where A is an attention matrix having the above-described attention vector a as rows. I is an identity matrix. The attention matrix A and the identity matrix I are processed as in Equation 2 above according to the Frobenius norm F. The variable P of the second term has a hyperparameter capable of encouraging diversity of the attention vector a and arbitrarily setting the attention vector a, as a coefficient. That is, the loss function includes the product of the second term and the coefficient.

The artificial neural network is trained by being updated such that the parameters of the artificial neural network are optimized. A method of optimizing the parameters may include, for example, Adaptive Moment Estimation (ADAM), Momentum, Nesterov Accelerated Gradient (NAG), Adaptive Gradient (Adagrad), RMSProp, and various gradient descent methods.

The artificial neural network may be further trained for the hyperparameters of the artificial neural network. In an embodiment, the hyperparameter to be learned may include at least one of the size of the contextual embedding vector c, the number of GRU-based hidden layers, the size of a hidden state vector, the size of a hidden layer of an attention unit da, the number of fully connected (FC) layers shared, the number of units (e.g., nodes) in each FC layer, an initial learning rate, a learning rate reduction factor, a dropout probability, a batch size, and a coefficient of the penalty term (P).

A method of learning the hyperparameters may include, for example, a tree-structured Parzen estimation method, but is not limited thereto. In one example, the hyperparameters described above may be optimized using Parzen estimation hundreds of times (approximately 500 times) for the artificial neural network of FIG. 2 or FIG. 3.

The artificial neural network may have relatively high performance even when trained using a smaller training data set. A task indicating needs of a patient may be classified into multiple groups, but there is a correlation between groups (e.g., a main task, a first auxiliary task, or a second auxiliary task). The artificial neural network uses the intermediate output of the shared network to determine multiple types of tasks, enabling efficient training.

Additionally, the decision model D may be configured and trained to find out the results of diagnosis and future treatment positioning of a patient, in addition to prediction of needs described above. In this case, the decision model D may be configured and trained to have functions of multi-categories prediction/classification tasks for predicting needs of a patient as described above and finding out the results of diagnosis and future treatment positioning of a patient.

It will be apparent to a person skilled in the art that the prediction system 1 may include other components not described herein. For example, the prediction system 1 may include other hardware components necessary for the operation described herein, including a network interface, an input device for data entry, and an output device for display, printing, or other data display.

A method of predicting needs of a patient for hospital resources according to another aspect of the present invention may be performed by a computing device including a processor (e.g., the system 1 of FIG. 1). Hereinafter, for clarity of description, the present invention will be described in more detail based on embodiments carried out by the system 1 of FIG. 1.

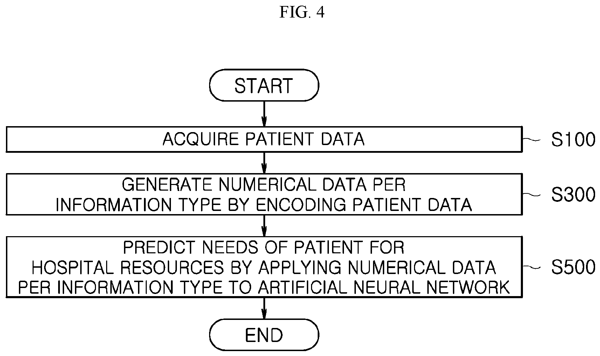

FIG. 4 is a flowchart of a method for predicting needs of a patient for hospital resources according to an embodiment of the present invention.

Referring to FIG. 4, the method of predicting patient needs for hospital resources includes a step S100 of acquiring patient data. The patient data includes structured data and natural language data. The structured data includes one or more of demographic information of a patient and measurement information of the patient. The demographic information includes one or more of gender and age. The measurement information includes measurement values for one or more measurement items among a pupil state, a systolic blood pressure (SBP), a diastolic blood pressure (DBP), a pulse, a respiration rate, a body temperature, a consciousness level, and an initial $O_2$ saturation. The natural language data includes current disease information of the patient as a first type of information. In addition, as a second type of information, the natural language data may include one or more of main symptom-related information, injury-related information, and history-related information.

In addition, the method of predicting needs of a patient for hospital resources includes a step S300 of encoding the patient data. Natural language data and structured data in patient data recorded in language and digits are converted into numerical data (S300). The numerical data may be generated per information type.

The step S300 includes converting the measurement information into numerical data through natural language processing.

In addition, a measurement matrix (or vector) for a patient may be formed using the numerical data of the measurement information (S300). The encoding result of the numerical data may be calculated as a matrix (or vector).

The step S300 includes converting the natural language data into numerical data by performing the natural language processing on the natural language data. The step S300 includes calculating a first type of natural language embedding vector by embedding text data of current disease information obtained through the natural language processing, and calculating a second type of natural language embedding vector by embedding text data of the remaining information of the natural language data obtained through natural language processing.

In addition, the step S300 may include converting the demographic information expressed in text into numerical data by natural language processing. By natural language processing of the categorical data, the categorical data is converted into text data, and an embedding vector of the text data is calculated (S300).

In an embodiment, the step S300 may include identifying text data of natural language data per information type. For example, HPI is identified as a first type of natural language information. The numerical data of the identified text data, that is, an embedding vector, is associated with the identified type data.

In addition, in the step S300, the first type of natural language embedding vector may be calculated per word. The first type of natural language data is textualized in word units, and an embedding vector of each word is calculated.

The step S300 may include forming a contextual embedding vector based on the first type of natural language embedding vector, the second type of natural language embedding vector, and the embedding vector of the demographic information. The second type of information among the natural language data and the numerical data of the categorical data are used to form a single embedding vector. For example, the second type of natural language embedding vector and the embedding vector of categorical data may be combined to form a contextual embedding vector c.

Through the preprocessing process described above, patient data may be converted into numerical data and applied to artificial neural networks.

The method of predicting needs of a patient for hospital resources includes a step S500 of predicting needs of a patient needs for hospital resources by applying the numerical data per information type to an artificial neural network. Data obtained by encoding the patient data (e.g., embedding vectors of words in sentences, etc.) is applied to the artificial neural network (S500).

The artificial neural network may include: an embedding model for calculating an embedding matrix of the patient data based on the numerical data; and a decision model for determining a task to which the patient data belongs by receiving at least a portion (e.g., measurement matrix) of an output value of the embedding model (e.g., embedding vector, embedding matrix, hidden state vector, etc.) and/or preprocessed numerical data. The decision model may determine multiple tasks to which a corresponding patient belongs.

In an embodiment, the decision model D may be configured to perform multiple binary classification for classifying whether a patient belongs to a plurality of task classes included in a corresponding task (e.g., a main task, a first auxiliary task or a second auxiliary task) to determine a main task, a first auxiliary task and/or a second auxiliary task.

For a main task including one or more task classes, e.g., hospital admission, endotracheal intubation, mechanical ventilation, vasopressor infusion, cardiac catheterization, surgery, intensive care unit (ICU) admission, and heart attack, a multiple binary classification operation may be performed, which determines the main task by determining whether a patient belongs to any task class.

Alternatively, a multiple binary classification operation for determining the first auxiliary task may be performed by determining a code corresponding to a diagnostic disease name recorded in the patient data of step S 100.

Alternatively, a multiple binary classification operation for determining the second auxiliary task may be performed by determining any one task class among hospital discharge, hospital admission, intensive care unit admission, transfer, and death.

The decision model D may predict the needs of a patient corresponding to the main task, the first auxiliary task, and/or the second auxiliary task associated with a corresponding patient through the multiple binary classification operation.

An input data set including a first type of natural language embedding vector e, a contextual embedding vector c, and a measurement matrix (or vector) is input to the pre-trained artificial neural network (S500). The artificial neural network used in step S500 may be the artificial neural network of FIG. 2 or FIG. 3.

In an embodiment, the artificial neural network includes an embedding model E and a decision model D, and the step S500 includes a step S510 of inputting the first type of natural language embedding vector e to the embedding model E or inputting the first type of natural language embedding vector e and the contextual embedding vector c into the embedding model E to calculate an embedding matrix of patient data.

When the artificial neural network of FIG. 2 is used, the first type of natural language embedding vector e is input to the embedding model E. When a first type of information consists of a plurality of words, each of a plurality of word embedding vectors may be sequentially input to the embedding model E in which the contextual embedding vector c is specified as an initial hidden state, as shown in FIG. 2. Then, an output matrix H including elements in which the contextual embedding vector c is overwritten per first type of natural language embedding vector e is formed.

When the artificial neural network of FIG. 3 is used, the first type of natural language embedding vector e is combined with an embedding vector before input, and is input to the embedding model E. When a plurality of word embedding vectors is input to the artificial neural network, the contextual embedding vector is combined with each of the plurality of word embedding vectors. The embedding model E of FIG. 3 forms an output matrix H by calculating an initial hidden state vector based on the word embedding vectors and the contextual embedding vector.

When a bidirectional-GRU-based hidden layer is configured to perform rolling processing, the contextual embedding vector of FIG. 3 may be provided to the bidirectional-GRU-based hidden layer together with each new input vector to be rolled. Then, the information of the contextual embedding vector encoded in the initial hidden state is subjected to the unrolling process in the bidirectional-GRU-based hidden layer, resulting in no degradation.

The output matrix H is used for predicting needs as the sentence embedding matrix M, or the sentence embedding matrix M based on the output matrix H and the attention matrix A is used for predicting needs.

The step S500 includes a step S550 of predicting needs of a patient by inputting a value output from the embedding model E and numerical data of the structured data, or a value output from the embedding model E to the decision model D.

The value output from the embedding model E includes the sentence embedding matrix M. Also, in some embodiments, the value output from the embedding model E further includes a final hidden state vector.

The numerical data of the structured data may be a measurement matrix.

In order to determine the main task and/or the first auxiliary task in step S530, the value output from the embedding model E and the numerical data of structured data may be used. For example, as shown in FIG. 2 or FIG. 3, the sentence embedding matrix M, the final hidden state vector and the measurement matrix may be input to the shared network.

In order to determine the second auxiliary task in step S530, only the value output from the embedding model E may be used. For example, as shown in FIG. 2 or FIG. 3, the sentence embedding matrix M and the final hidden state vector may be input to the networks N4, N5, and N6.

The artificial neural network is pre-trained so that the decision model D determines multiple tasks using the intermediate data set of a training patient, and has parameters and/or hyperparameters trained in advance to determine a task corresponding to needs of a patient needs based on input data. As the training, internal structure, and processing operation of the artificial neural network have been described above, a detailed description thereof will be omitted.

The present invention may predict needs for multiple treatments of a patient, which cannot generally be predicted as a single result in many medical emergency situations through the artificial neural network.

In addition, the artificial neural network may perform various predictions by using features scattered in other unstructured data formats (e.g., language) through natural language processing. That is, a prediction operation may be performed using information on both structured data and unstructured data through natural language processing.

[Examples]

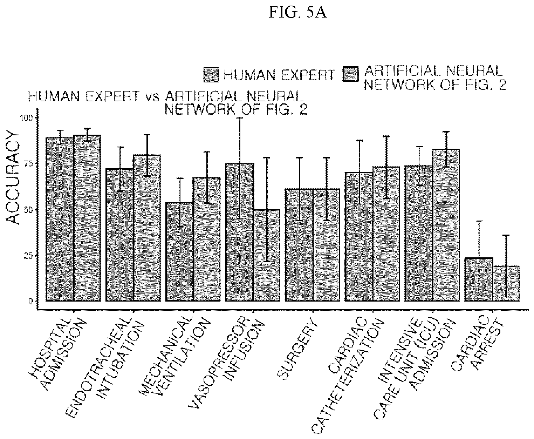

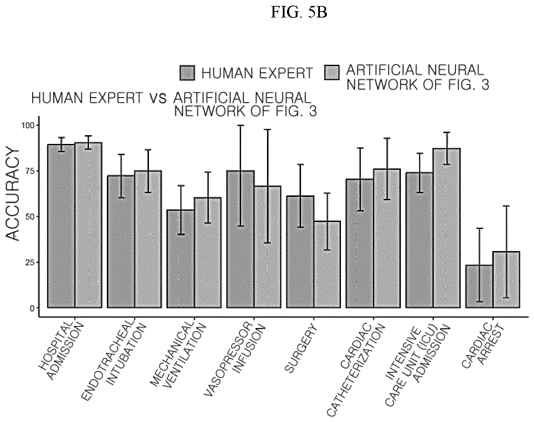

FIGS. 5 to 6 are diagrams for evaluating performance of predicting needs of a patient using an artificial neural network, according to an experimental example of the present invention.

In the above experimental example, approximately 4,2000 pieces of patient data were used for validation. The artificial neural network is configured to determine one main task, a first auxiliary task and a second auxiliary task. The main tasks include hospital admission, endotracheal intubation, mechanical ventilation (MV), vasopressor infusion, cardiac catheterization (CAG), surgery, intensive care unit (ICU) admission, and cardiac arrest within 24 hours after emergency room (ED) arrival. The first auxiliary task includes an emergency room diagnosis disease name code. The second auxiliary task includes five components: hospital discharge, ward admission, intensive care unit admission, operating room (OR) transfer, or death. That is, the artificial neural network includes one main task and five auxiliary tasks (one first auxiliary task and four second auxiliary tasks).

In the above experimental example, the quality of attention mapping was evaluated with respect to 100 random samples of patient data by an artificial neural network and another human expert with 2 years of experience as an emergency room medical service (EMS) director.

To evaluate the quality of attention mapping, a 5-point Likert scale technique was used. The patterns of attention mapping are rated with 5 levels in terms of clinical relevance.

FIG. 5A is a diagram comparing the artificial neural network of FIG. 2 and a human expert in prediction performance, and FIG. 5B is a diagram comparing the artificial neural network of FIG. 3 and a human expert in prediction performance.

Referring to FIGS. 5A and 5B, the artificial neural network has a level of performance similar to the evaluation result of a human expert. In particular, the artificial neural network has better performance than human experts in predicting needs for mechanical ventilation (MV) and ICU admission.

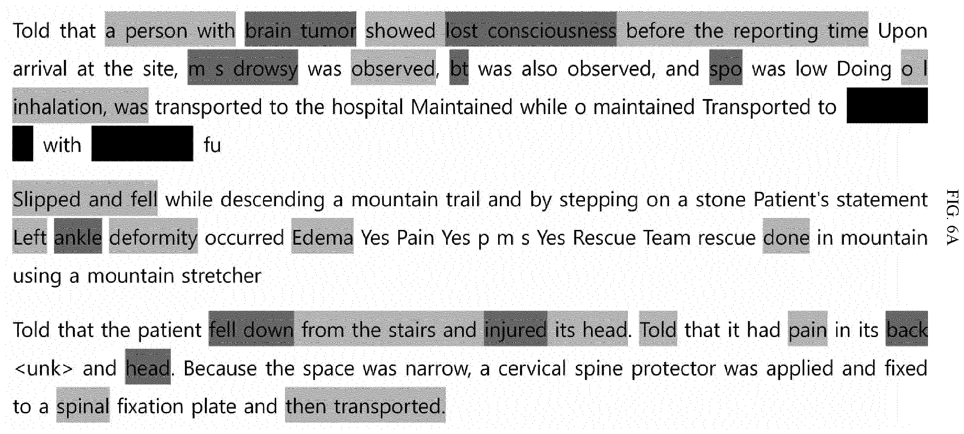

FIGS. 6A to 6D are diagrams illustrating samples of an attention map selected from a range of level 3 in the order of the highest level among 5 levels. A result of attention mapping of data of the patient data by the artificial neural network may be visualized on the patient data. In the experimental example, the result of the attention mapping is visualized through a gradient-weighted class activation map (Grad-CAM).

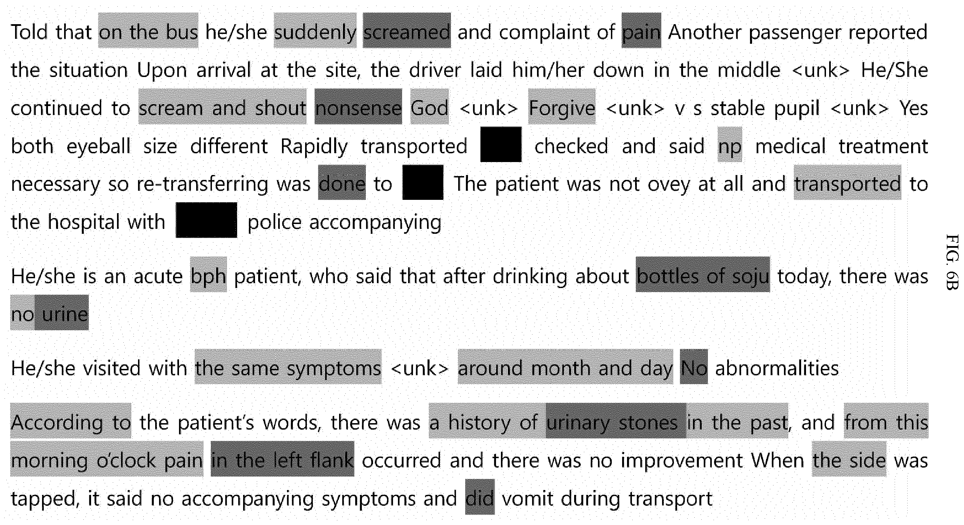

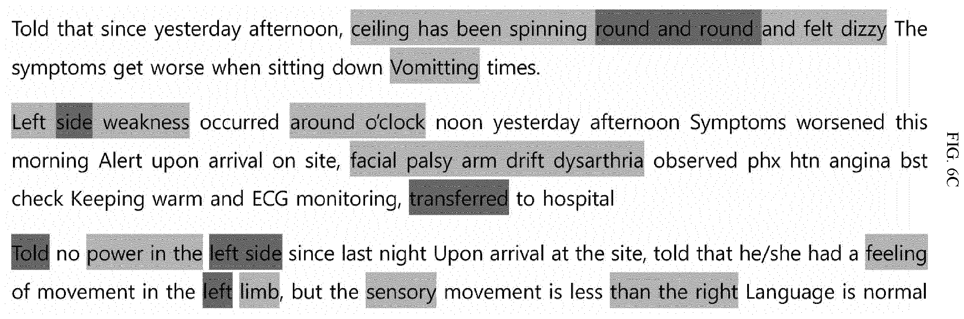

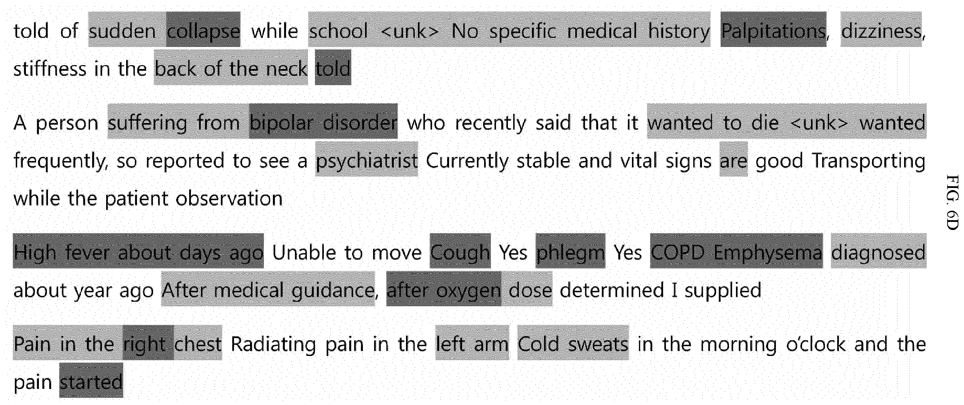

When each sentence shown in FIGS. 6Ato 6D is input as patient data, the needs of a patient are predicted by determining a task to which an input sentence belongs. Here, display may be performed to pay more attention to an input word related to a task to be predicted. It is confirmed that the artificial neural network has ability to focus on the same or similar word as the words which humans practically focus on in order to predict the needs of a patient.

The method for predicting needs of a patient for hospital resources according to the embodiments described above and the operation by the system 1 for performing the same may be at least partially implemented as a computer program and recorded in a computer-readable recording medium. For example, the method and the operation may be embodied with a program product consisting of a computer-readable medium including program codes, which may be executed by a processor for performing any or all steps, operations, or processes described.

The computer-readable recording medium includes all kinds of recording devices in which data readable by a computer is stored. Examples of the computer-readable recording medium include a read only memory (ROM), a Random Access Memory (RAM), a CD-ROM, a magnetic tape, a floppy disk, an optical data storage device, and the like. The computer-readable recording medium may be distributed over computer systems connected through networks so that the computer readable codes are stored and executed in a distributed fashion. In addition, functional programs, codes, and code segments for implementing the present embodiment may be easily understood by those skilled in the art to which the present embodiment belongs.

Although the present invention as described above has been described with reference to the embodiments shown in the drawings, it will be understood that the embodiments are merely exemplary, and that various modifications and variations of the embodiments are possible therefrom by those of ordinary skill in the art. However, such modifications should be considered to be within the technical protection scope of the present invention. Accordingly, the technical scope of the present invention should be defined by the accompanying claims.

**Industrial Applicability**

The present invention may efficiently predict needs of a patient for hospital resources using an artificial neural network trained by machine learning, one of the 4th industrial technologies, so that it is expected to have high industrial applicability in the medical field.

**Claims**

1. A method for predicting needs of a patient for hospital resources, the method being performed by a processor, the method comprising:

generating numerical data per information type by encoding natural language data and structured data in patient data recorded in language and digits; and
predicting a task corresponding to the needs of the patient for hospital resources by applying the numerical data per information type to an artificial neural network,
wherein the artificial neural network comprises an embedding model for calculating an embedding matrix of the patient data based on at least a part of the numerical data; and
a decision model for determining the task to which the patient data belongs by receiving the embedding matrix of the patient data, or the embedding matrix of the patient data and the numerical data of the structured data.

2. The method according to claim 1, wherein the natural language data comprises current disease information of the patient, and the structured data comprises at least one of demographic information of the patient and measurement information of the patient,
wherein the generating of the numerical data per information type comprises:

performing natural language processing on the natural language data;
calculating a first type of natural language embedding vector by embedding text data of the current disease information obtained through the natural language processing;
calculating a second type of natural language embedding vector by embedding text data of remaining information of the natural language data obtained through the natural language processing;
performing natural language processing on the structured data; and
calculating an embedding vector of the demographic information by embedding the text data of the demographic information obtained through the natural language processing, or performing conversion into the numerical data through the natural language processing.

3. The method according to claim 2, wherein the predicting of the task corresponding to the needs of the patient for the hospital resources comprises

calculating, by the embedding model, an embedding matrix of the patient data from the first type of natural language embedding vector and a contextual embedding vector,

wherein the contextual embedding vector is based on the second type of natural language embedding vector and the embedding vector of the demographic information, and

wherein the embedding model comprises a unidirectional or bidirectional gated recurrent unit (GRU)-based hidden layer that extracts features of input data and calculates a hidden state vector; and an attention layer that receives an output matrix of the hidden layer and calculates the embedding matrix of the patient data.

4. The method according to claim 3, wherein an initial hidden state of the embedding model is specified as the contextual embedding vector,

wherein the predicting of the task corresponding to the needs of the patient for the hospital resources comprises inputting the first type of natural language embedding vector into the initial hidden layer of the embedding model.

5. The method according to claim 4, further comprising: when a plurality of first types of natural language embedding vectors are input to the embedding model, sequentially inputting the plurality of first types of natural language embedding vectors to the hidden layer.

6. The method according to claim 3, wherein the predicting of the task corresponding to the needs of the patient for the hospital resources comprises inputting a combined vector obtained by combining the first type of natural language embedding vector with the contextual embedding vector into the initial hidden layer of the embedding model.

7. The method according to claim 3, wherein the predicting of the task corresponding to the needs of the patient for the hospital resources comprises forming a hidden matrix H consisting of final hidden state vectors, and

wherein the attention layer calculates the embedding matrix M of the patient data based on the hidden matrix H and the attention matrix A based on an attention weight.

8. The method according to claim 3, wherein the predicting of the task corresponding to the needs of the patient for the hospital resources includes receiving, by the decision model, at least the embedding matrix of the patient data among the embedding matrix of the patient data, a final hidden state vector, and the numerical data of the measurement information, and

wherein the decision model is a fully connected layer composed of two or more layers.

9. The method according to claim 8, wherein the artificial neural network is pre-trained such that the decision model determines at least one task among multiple tasks using a training data set for a plurality of training patients, and

wherein the training data set consists of training samples for each training patient, and each training sample comprises at least an embedding matrix of patient data among the embedding matrix of the patient data, a final hidden state vector, and numerical data of measurement information for a corresponding training patient.

10. The method according to claim 8, wherein the decision model is trained to perform multiple binary classification for determining a task class to which the patient data belongs among a plurality of task classes included in a corresponding task to determine at least one task among multiple tasks.

11. The method according to claim 8, wherein the fully connected layer comprises one or more of a first network for determining a main task, a second network for determining a first auxiliary task, and a third network for determining a second auxiliary task,

wherein the first network or the second network is configured to receive the embedding matrix of the patient data and the final hidden state vector, and

wherein the third network is configured to receive the embedding matrix of the patient data, the final hidden state vector, and the numerical data of the measurement information.

12. The method according to claim 11, wherein a loss function of the artificial neural network comprises:

a term indicating a weighted sum of a cross entropy loss function between networks per task of the fully connected layer, and

another term obtained by applying the Frobenius norm to an attention matrix, a transform matrix of the attention matrix, and an identity matrix.

13. The method according to claim 2, wherein the natural language data further includes one or more of main symptom-related information, injury-related information, and history-related information, and

wherein the demographic information comprises one or more information among gender and age.

14. The method according to claim 2, wherein the measurement information includes measurement values for one or more measurement items among a pupil state, a systolic blood pressure (SBP), a diastolic blood pressure (DBP), a pulse, a respiration rate, a body temperature, a consciousness level, and an

initial $O_2$ saturation.

15. The method according to claim 11, wherein the main task comprises one or more of hospital admission, endotracheal intubation, mechanical ventilation, vasopressor infusion, cardiac catheterization, surgery, intensive care unit (ICU) admission, and cardiac arrest as a task class,

    wherein the first auxiliary task comprises an emergency room diagnosis disease name code as a task class, and
    wherein the second auxiliary task comprises one or more of discharge, ward admission, intensive care unit (ICU) admission, transfer, and death as a task class.

16. A computer-readable recording medium having recorded thereon a computer program for performing the method of predicting needs of a patient according to any one of claims 1 to 15.

17. A system comprising:

    a data acquisition device configured to acquire patient data comprising natural language data describing a condition of a patient and structured data, the patent data being recorded in language and digits;
    an encoding module configured to generate numerical data per information type by encoding the natural language data and the structured data in the patient data; and
    a prediction module configured to predict a task corresponding to needs of the patient for hospital resources by applying the numerical data to an artificial neural network,
    wherein the artificial neural network comprises:

        an embedding model for calculating an embedding matrix of the patient data based on at least a part of the numerical data; and
        a decision model for determining the task to which the patient data belongs by receiving the embedding matrix of the patient data, or the embedding matrix of the patient data and the numerical data of the structured data.

18. The system according to claim 17, wherein the natural language data comprises current disease information of the patient, and the structured data comprises at least one of demographic information of the patient and measurement information of the patient, wherein the encoding module is configured to:

    perform natural language processing on the natural language data,

    calculate a first type of natural language embedding vector by embedding text data of the current disease information obtained through the natural language processing,
    calculate a second type of natural language embedding vector by embedding text data of remaining information of the natural language data obtained through the natural language processing,
    perform natural language processing on the structured data; and
    calculate an embedding vector of the demographic information by embedding the text data of the demographic information obtained through the natural language processing, or performing conversion into the numerical data through the natural language processing.

19. The system according to claim 18, wherein the prediction module is configured to calculate, by the embedding model, an embedding matrix of the patient data from the first type of natural language embedding vector and a contextual embedding vector,

    wherein the contextual embedding vector is based on the second type of natural language embedding vector and the embedding vector of the demographic information, and
    wherein the embedding model comprises a unidirectional or a bidirectional gated recurrent unit (GRU)-based hidden layer that extracts features of input data and calculates a hidden state vector; and an attention layer that receives an output matrix of the hidden layer and calculates an embedding matrix of the patient data.

20. The system according to claim 18, wherein an initial hidden state of the embedding model is specified as the contextual embedding vector, and wherein the prediction module is configured to input the first type of natural language embedding vector to the initial hidden layer of the embedding model.

21. The system according to claim 18, wherein the prediction module is configured to input a combined vector obtained by combining the first type of natural language embedding vector with the contextual embedding vector into the initial hidden layer of the embedding model.

22. The system according to claim 18, wherein the prediction module is configured to input, to the decision model, at least the embedding matrix of the patient data among the embedding matrix of the patient data, a final hidden state vector, and the numerical data of the measurement information.

23. The system according to claim 17, further comprising:

a training module configured to train the artificial neural network such that the decision model determines at least one task among multiple tasks using an intermediate data set for training patients,

wherein the training data set consists of training samples for each training patient, and each training sample comprises at least an embedding matrix of patient data among the embedding matrix of the patient data, a final hidden state vector, and numerical data of measurement information for a corresponding training patient.

FIG. 1

<u>1</u>

| DATA ACQUISITION DEVICE | ~ 10 |
| ENCODING MODULE | ~ 30 |
| PREDICTION MODULE | ~ 50 |
| TRAINING MODULE | ~ 70 |

FIG. 2

FIG. 3

FIG. 4

```
                    ┌──────────────┐
                    │    START     │
                    └──────┬───────┘
                           │
 ┌─────────────────────────────────────────────────────┐
 │             ACQUIRE PATIENT DATA                     │ ─── S100
 └─────────────────────────┬───────────────────────────┘
                           │
 ┌─────────────────────────────────────────────────────┐
 │           GENERATE NUMERICAL DATA PER                │
 │    INFORMATION TYPE BY ENCODING PATIENT DATA         │ ─── S300
 └─────────────────────────┬───────────────────────────┘
                           │
 ┌─────────────────────────────────────────────────────┐
 │           PREDICT NEEDS OF PATIENT FOR              │
 │   HOSPITAL RESOURCES BY APPLYING NUMERICAL DATA      │ ─── S500
 │ PER INFORMATION TYPE TO ARTIFICIAL NEURAL NETWORK    │
 └─────────────────────────┬───────────────────────────┘
                           │
                    ┌──────┴───────┐
                    │     END      │
                    └──────────────┘
```

FIG. 5A

FIG. 5B

HUMAN EXPERT vs ARTIFICIAL NEURAL NETWORK OF FIG. 3

Told that a person with brain tumor showed lost consciousness before the reporting time Upon arrival at the site, m s drowsy was observed, bt was also observed, and spo was low Doing o l inhalation, was transported to the hospital Maintained while o maintained Transported to ███ with ████ fu

Slipped and fell while descending a mountain trail and by stepping on a stone Patient's statement Left ankle deformity occurred Edema Yes Pain Yes p m s Yes Rescue Team rescue done in mountain using a mountain stretcher

Told that the patient fell down from the stairs and injured its head. Told that it had pain in its back <unk> and head. Because the space was narrow, a cervical spine protector was applied and fixed to a spinal fixation plate and then transported.

FIG. 6A

EP 4 156 202 A1

Told that on the bus he/she suddenly screamed and complaint of pain Another passenger reported the situation Upon arrival at the site, the driver laid him/her down in the middle <unk> He/She continued to scream and shout nonsense God <unk> Forgive <unk> v s stable pupil <unk> Yes both eyeball size different Rapidly transported ███ checked and said np medical treatment necessary so re-transferring was done to ███ The patient was not ovey at all and transported to the hospital with ███ police accompanying

He/she is an acute bph patient, who said that after drinking about bottles of soju today, there was no urine

He/she visited with the same symptoms <unk> around month and day No abnormalities

According to the patient's words, there was a history of urinary stones in the past, and from this morning o'clock pain in the left flank occurred and there was no improvement When the side was tapped, it said no accompanying symptoms and did vomit during transport

EP 4 156 202 A1

FIG. 6B

Told that since yesterday afternoon, ceiling has been spinning round and round and felt dizzy The symptoms get worse when sitting down Vomitting times.

Left side weakness occurred around o'clock noon yesterday afternoon Symptoms worsened this morning Alert upon arrival on site, facial palsy arm drift dysarthria observed phx htn angina bst check Keeping warm and ECG monitoring, transferred to hospital

Told no power in the left side since last night Upon arrival at the site, told that he/she had a feeling of movement in the left limb, but the sensory movement is less than the right Language is normal

FIG. 6C

told of sudden collapse while school <unk> No specific medical history Palpitations, dizziness, stiffness in the back of the neck told

A person suffering from bipolar disorder who recently said that it wanted to die <unk> wanted frequently, so reported to see a psychiatrist Currently stable and vital signs are good Transporting while the patient observation

High fever about days ago Unable to move Cough Yes phlegm Yes COPD Emphysema diagnosed about year ago After medical guidance, after oxygen dose determined I supplied

Pain in the right chest Radiating pain in the left arm Cold sweats in the morning o'clock and the pain started

FIG. 6D

## INTERNATIONAL SEARCH REPORT

| International application No. |
|---|
| **PCT/KR2021/006287** |

**A. CLASSIFICATION OF SUBJECT MATTER**

**G16H 40/20**(2018.01)i; **G06Q 10/06**(2012.01)i; **G06N 20/00**(2019.01)i; **G06N 3/08**(2006.01)i

According to International Patent Classification (IPC) or to both national classification and IPC

**B. FIELDS SEARCHED**

Minimum documentation searched (classification system followed by classification symbols)

G16H 40/20(2018.01); A61B 5/00(2006.01); A61B 5/0468(2006.01); G06F 17/27(2006.01); G06F 17/28(2006.01); G06F 19/00(2011.01); G06N 3/04(2006.01); G06N 3/08(2006.01); G16H 50/20(2018.01); G16H 50/70(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 환자(patient), 인코딩(encoding), 임베딩(embedding), 행렬(matrix), 인공신경망 (artificial neural network)

**C. DOCUMENTS CONSIDERED TO BE RELEVANT**

| Category* | Citation of document, with indication, where appropriate, of the relevant passages | Relevant to claim No. |
|---|---|---|
| A | US 2019-0287684 A1 (VVC HOLDING CORPORATION) 19 September 2019 (2019-09-19) See paragraphs [0037]-[0052] and claims 1 and 10. | 1-23 |
| A | US 2018-0365560 A1 (INTERNATIONAL BUSINESS MACHINES CORPORATION) 20 December 2018 (2018-12-20) See paragraphs [0036]-[0047] and claims 1, 2 and 4. | 1-23 |
| A | JP 2019-185748 A (BAIDU USA L.L.C.) 24 October 2019 (2019-10-24) See paragraph [0046] and claim 1. | 1-23 |
| A | US 2019-0133480 A1 (KONINKLIJKE PHILIPS N.V.) 09 May 2019 (2019-05-09) See paragraphs [0027]-[0031] and claims 1 and 2. | 1-23 |
| A | US 2017-0293725 A1 (SIEMENS HEALTHCARE GMBH) 12 October 2017 (2017-10-12) See paragraphs [0018]-[0025]. | 1-23 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **30 August 2021** | **30 August 2021** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
**Information on patent family members**

International application No.

**PCT/KR2021/006287**

| Patent document cited in search report | | | Publication date (day/month/year) | Patent family member(s) | | | Publication date (day/month/year) |
|---|---|---|---|---|---|---|---|
| US | 2019-0287684 | A1 | 19 September 2019 | US | 10784000 | B2 | 22 September 2020 |
| | | | | US | 2019-0287685 | A1 | 19 September 2019 |
| US | 2018-0365560 | A1 | 20 December 2018 | | None | | |
| JP | 2019-185748 | A | 24 October 2019 | CN | 110379225 | A | 25 October 2019 |
| | | | | JP | 6722789 | B2 | 15 July 2020 |
| | | | | US | 1074829 | B2 | 27 July 2021 |
| | | | | US | 2019-0318648 | A1 | 17 October 2019 |
| US | 2019-0133480 | A1 | 09 May 2019 | WO | 2019-091990 | A1 | 16 May 2019 |
| US | 2017-0293725 | A1 | 12 October 2017 | CN | 107292086 | A | 24 October 2017 |
| | | | | EP | 3229157 | A1 | 11 October 2017 |
| | | | | US | 9984772 | B2 | 29 May 2018 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 1020090001551 A